# EUROPEAN PATENT SPECIFICATION

(11) **EP 4 441 247 B1**
(45) Date of publication and mention of the grant of the patent: **31.12.2025**
(21) Application number: 22814539.7
(22) Date of filing: 28.11.2022
(51) Int. Cl.: C12Q 1/66, C12Q 1/682

(54) **LUCIFERASE-INTERCALATING DYE CONJUGATES FOR RATIOMETRIC NUCLEIC ACID DETECTION**
LUCIFERASE-INTERKALIERENDE FARBSTOFFKONJUGATE ZUM RATIOMETRISCHEN NUKLEINSÄURENACHWEIS
CONJUGUÉS DE COLORANTS INTERCALAIRES DE LA LUCIFÉRASE POUR LA DÉTECTION RATIOMÉTRIQUE D'ACIDES NUCLÉIQUES

(30) Priority: 29.11.2021 NL 2029944
(43) Date of publication of application: 09.10.2024
(73) Proprietor: Technische Universiteit Eindhoven, 5612 AE Eindhoven (NL)
(72) Inventor: DE STIGTER, Yosta, 5612 AE Eindhoven (NL); ROSIER, Bas, 5612 AE Eindhoven (NL); MERKX, Maarten, 5612 AE Eindhoven (NL)
(74) Representative: Algemeen Octrooi- en Merkenbureau B.V.
(86) International application number: PCT/NL2022/050685
(87) International publication number: WO 2023/096493

(56) References cited:
- YOSHIDA WATARU ET AL: "Detection of Histone Modification by Chromatin Immunoprecipitation Combined Zinc Finger Luciferase-Based Bioluminescence Resonance Energy Transfer Assay", ANALYTICAL CHEMISTRY, vol. 85, no. 13, 14 June 2013 (2013-06-14), US, pages 6485 - 6490, XP055943137, ISSN: 0003-2700, DOI: 10.1021/ac401036k
- ENGELEN WOUTER ET AL: "Firefly Luciferase-based Fusion Proteins and their Applications in Bioanalysis", CHEMICAL COMMUNICATIONS, vol. 53, no. 19, 1 January 2017 (2017-01-01), UK, pages 2862 - 2865, XP055854506, ISSN: 1359-7345, DOI: 10.1039/C6CC10032E
- SMIRNOVA DARIA V. ET AL: "Firefly Luciferase-based Fusion Proteins and their Applications in Bioanalysis", vol. 93, no. 2, 30 November 2016 (2016-11-30), US, pages 436 - 447, XP055865555, ISSN: 0031-8655, Retrieved from the Internet <URL:https://api.wiley.com/onlinelibrary/tdm/v1/articles/10.1111%2Fphp.12656> DOI: 10.1111/php.12656
- F. X. SCHAUB ET AL: "Fluorophore-NanoLuc BRET Reporters Enable Sensitive In Vivo Optical Imaging and Flow Cytometry for Monitoring Tumorigenesis", CANCER RESEARCH, vol. 75, no. 23, 30 September 2015 (2015-09-30), US, pages 5023 - 5033, XP055741027, ISSN: 0008-5472, DOI: 10.1158/0008-5472.CAN-14-3538

## Description

### Field of the invention

The invention relates probes for detecting and/or quantifying double-stranded DNA. The invention particularly relates to luminescence based intercalating probes, and methods of using such probes to detect or quantify double-stranded DNA.

### Introduction

Detection of DNA and RNA is fundamental for diagnosing and preventing infectious diseases caused by pathogens, such as viruses and bacteria. The benchmark for DNA detection is the polymerase chain reaction (PCR), which is based on the rapid generation of large amounts of identical copies from a single fragment of target DNA. PCR relies on the binding of short DNA primers that bind to the target DNA after which a DNA polymerase enzyme generates new DNA fragments based on the target template. To this end, PCR requires three distinct thermal cycling steps in which the temperature of the reaction mixture is varied between 50-98°C. To monitor and quantify the DNA during PCR, intercalating dye molecules are used that exhibit a large increase in fluorescence when binding to DNA. The fluorescent signal is generated using an optical excitation source such as a laser or LED, after which the intensity of the collected light is used as a measure for DNA concentration. Although quantitative PCR (qPCR) has been shown to allow highly sensitive detection of DNA, the required set-up can be prohibitive in situations where the use of expensive, specialized equipment (containing lasers, optics and a temperature controller) is limited or undesirable, for example in point-of-care diagnostic testing. Additionally, fluorescence suffers from a high background signal, which can pose a problem when performing measurements in complex media such as patient blood.

In order to reduce the complexity of PCR-based detection strategies and move towards point-of-care applications, several alternative approaches have been investigated. A well-established strategy is to use isothermal nucleic acid amplification methods that allow for amplification at constant temperatures, eliminating the need for thermal cycling equipment. Two examples that have received attention recently are the SHERLOCK and DETECTR platforms, that combine isothermal amplification with CRISPR-associated (Cas) proteins that are capable of recognizing highly specific DNA or RNA sequences (Gootenberg, J. S. et al. Nucleic acid detection with CRISPR-Cas13a/C2c2. Science. 356, 438-442 (2017); Chen, J. S. et al. CRISPR-Cas12a target binding unleashes indiscriminate single-stranded DNase activity. Science. 360, 436-439 (2018)). The SHERLOCK method uses isothermal amplification via recombinase polymerase amplification (RPA), which relies on a set of three enzymes for binding of primers and subsequent DNA amplification. It can be performed at a constant temperature of 37-42°C, obviating the need for thermal cycling. Similarly, DETECTR uses loop-mediated isothermal amplification (LAMP), which is performed at a constant temperature of 60-65°C and is based on a set of specifically designed looped primers that facilitate the amplification cycle. For both SHERLOCK and DETECTR, a fluorescent output signal is generated upon binding of the Cas proteins to single-stranded RNA and double-stranded DNA, respectively, and subsequent cleavage of single-stranded nucleic acid reporters containing a fluorophore and quencher pair. For point-of-care applications, a colorimetric alternative to the fluorescent readout was realized and incorporated into a lateral flow assay (Patchsung, M. et al. Clinical validation of a Cas13-based assay for the detection of SARS-CoV-2 RNA. Nat. Biomed. Eng. 4, 1140-1149 (2020)). Although these assays successfully combine isothermal amplification with specific and ultrasensitive detection of viral nucleic acid sources, they still rely on a fluorescent readout that requires an external excitation source or lateral flow strips, which typically only provide qualitative information.

Sensors based on bioluminescence represent a powerful alternative strategy to fluorescent detection. By employing bioluminescent luciferase enzymes that produce light without requiring external excitation, it is possible to circumvent high background signals and the use of specialized optical instrumentation for detection. Such bioluminescent proteins can be coupled to an acceptor to enable bioluminescent resonance energy transfer (BRET), similar to the more commonly used FRET-based approaches. This principle has already been employed to create analogues of fluorescent detection methods, including bioluminescent molecular beacons, which consist of a luciferase and acceptor dye connected to a DNA hairpin. The hairpin initially brings both components into close proximity, facilitating BRET, but separates the two in complexation with target ssDNA or ssRNA (Engelen, W., van de Wiel, K. M., Meijer, L. H. H., Saha, B. & Merkx, M. Nucleic acid detection using BRET-beacons based on bioluminescent protein-DNA hybrids. Chem. Commun. 53, 2862-2865 (2017)). Also, intercalating dyes have been used in combination with bioluminescent proteins, in which a luciferase was fused to a DNA-targeting zinc finger protein (Yoshida et al., Anal. Chem. 2013, 85, 13, 6485-6490). Upon the binding of the zinc finger to target dsDNA, the intercalating dye and luciferase protein are brought into proximity, leading to BRET from the luciferase to the dye. Unlike the intercalating dyes in qPCR, both these approaches focus on the specific detection of DNA and require chemical synthesis of new probes for every new target DNA.

To improve the sensitivity of point-of-care diagnostic applications, bioluminescence has also been combined with isothermal amplification of the target nucleic acid. The 'Bioluminescent Assay in Real Time' (BART) relies on LAMP amplification of target DNA, and enzymes that consume or convert one of the by-products of DNA polymerization to generate light (Gandelman, O. A. et al. Novel bioluminescent quantitative detection of nucleic acid amplification in real-time. PLoS One 5, (2010)). While this method has shown to be able to detect single copies of DNA, it suffers from auto-inhibition, which makes the output signal time-dependent and difficult to interpret. Furthermore, detection of specific DNA sequences has been explored in combination with isothermal amplification through split-luciferases conjugated to single-stranded DNA (ssDNA) (Chang, D., Kim, K. T., Lindberg, E. & Winssinger, N. Smartphone DNA or RNA Sensing Using Semisynthetic Luciferase-Based Logic Device. ACS Sensors 5, 807-813 (2020)). Here, repeating target sequences on rolling circle amplification (RCA) amplicons are recognized by the system, causing the tandem assembly of the split luciferases, which reconstitutes NanoLuc, leading to blue emission. Similar to the molecular beacons and zinc fingers, this strategy requires chemical synthesis of new probes for every new target DNA.

These problems, among others are overcome by the invention as described in the appended claims.

### Brief description of the figures

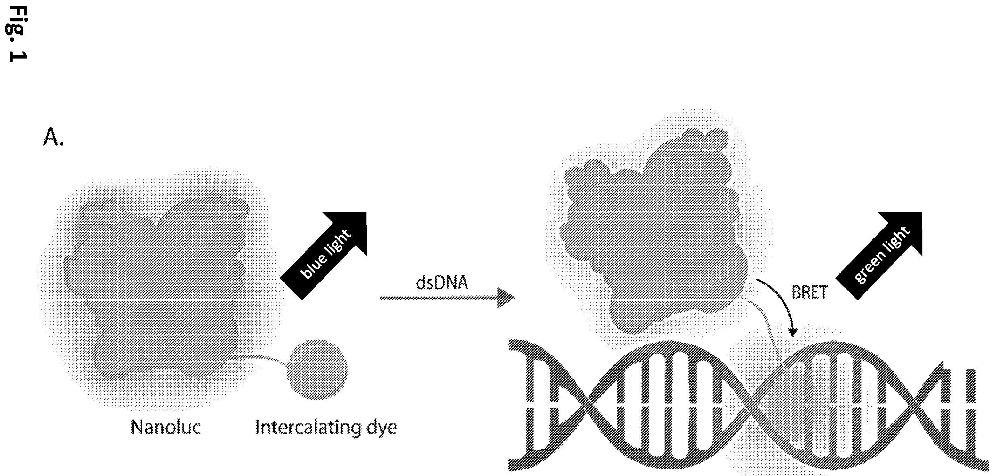

**Figure 1** **|** *Schematic of the bioluminescent intercalating dye. The probe consists of the NanoLuc luciferase (blue, λₘₐₓ ~ 460 nm) conjugated to an intercalating dye (green) through cysteine-maleimide chemistry. In absence of double-stranded (dsDNA), the intercalating dye will be minimally fluorescent, and the blue emission of NanoLuc can be witnessed (left). In presence of dsDNA, the intercalating dye binds to the dsDNA, and allows for BRET, leading to green emission (right).*

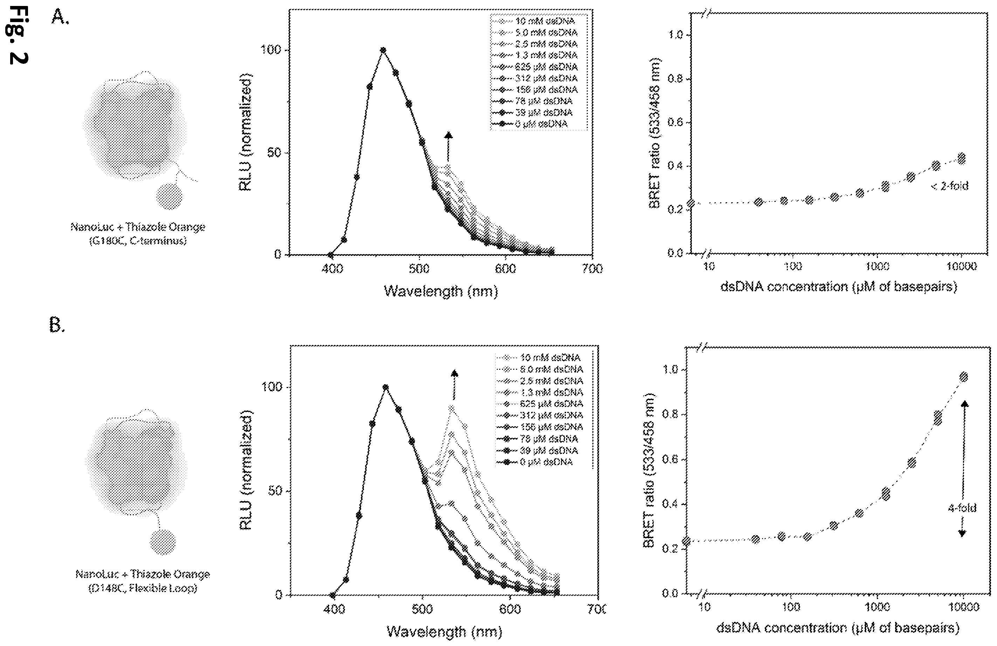

**Figure 2** | *(A) Bioluminescence titration with dsDNA of NanoLuc-Thiazole Orange (position* D148C, *flexible loop). (B) Bioluminescence titration with dsDNA of NanoLuc-Thiazole Orange (position G182C*, *C-terminus). For both variants: the conjugate (left, 1 nM) was added to a 2-fold dilution series of Salmon Sperm DNA ranging from (in base pairs of DNA) 39 µM-10, 000 µM. Incubation was performed at room temperature for 30 minute in 1xPBS + 1 mg*/*mL BSA,* 5% *DMSO, pH 7.4. NanoGlo substrate (1000x dilution) was added, after which the emission intensity in the range of 398-653 nm was measured.*

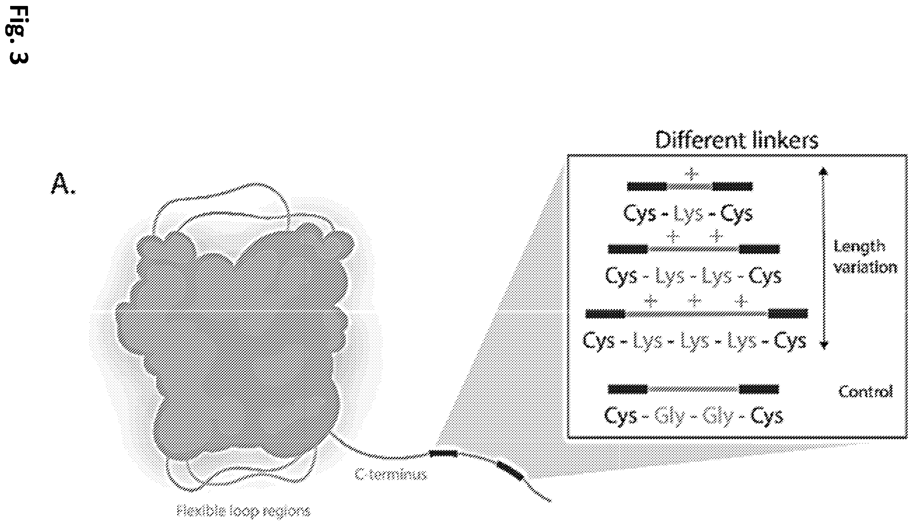

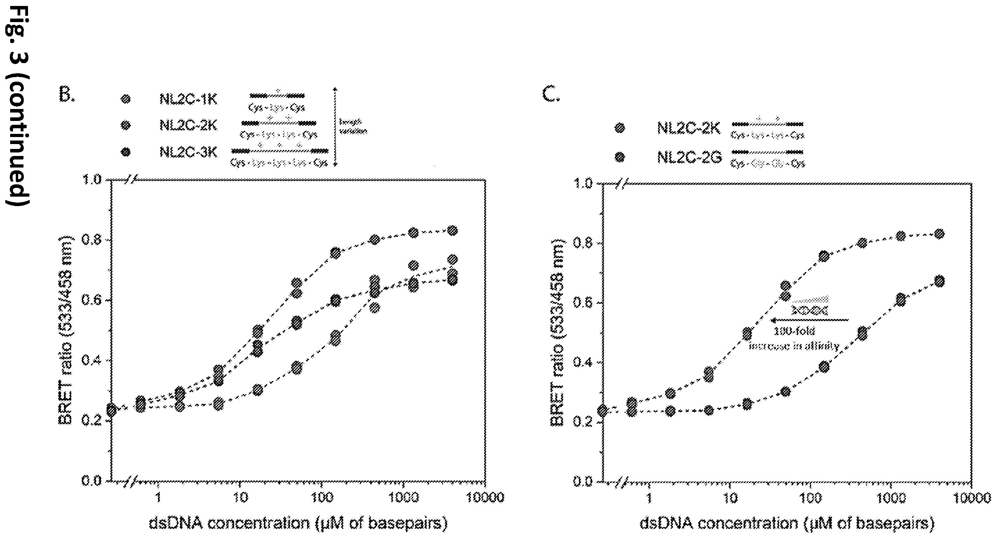

**Figure 3** **|** *(A) Schematic of the different linker variants that were incorporated at the C-terminus of NanoLuc, including 1 to 3 lysines and 2 glycines (negative control). (B) Bioluminescence titration with dsDNA of NanoLuc variant with two intercalating dyes and either 1 (NL2C-1K), 2 (NL2C-2K) or 3 (NL2C-3K) lysines in between (position G182*, *C-terminus). (C) Bioluminescence titration with dsDNA of NanoLuc variant with two intercalating dyes and either 2 lysines (NL2C-2K) or 2 glycines (NL2C-2G, negative control) in between (position G182*, *C-terminus). For all variants: the conjugate (1 nM) was added to a 2-fold dilution series of Salmon Sperm DNA ranging from (in base pairs of DNA) 0.76 µM-5, 000 µM. Incubation was performed at room temperature for 30 minute in 1xPBS + 1 mg*/*mL BSA,* 5% *DMSO, pH 7.4. NanoGlo substrate (1000x dilution) was added, after which the emission intensity in the range of 398-653 nm was measured.*

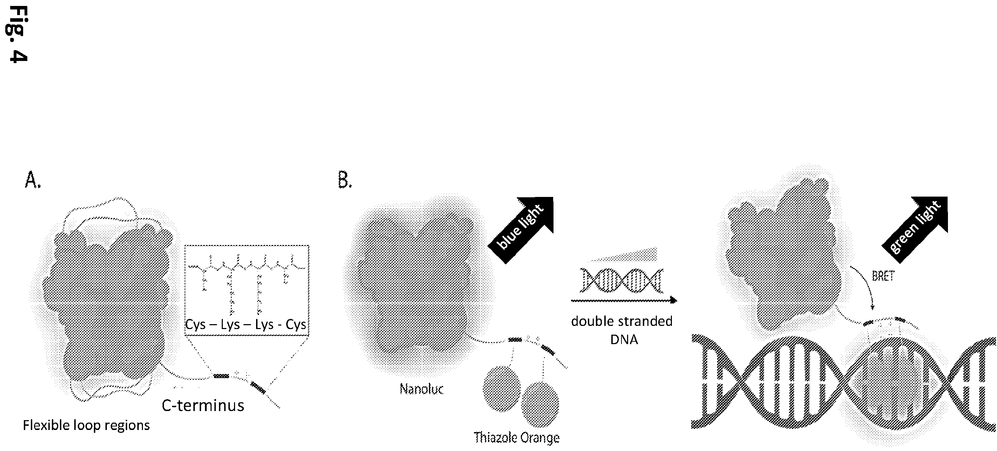

**Figure 4** | *(A) Schematic of the NanoLuc variant containing two cysteines separated by two positively charged lysine residues. (B) Schematic of the Nanoluc-2xTO probe with two dyes, which consists of the NanoLuc luciferase (blue) conjugated to two intercalating dyes (green) separated by a positively charged linker, which is expected to cause additional interactions with the negatively charged dsDNA backbone.*

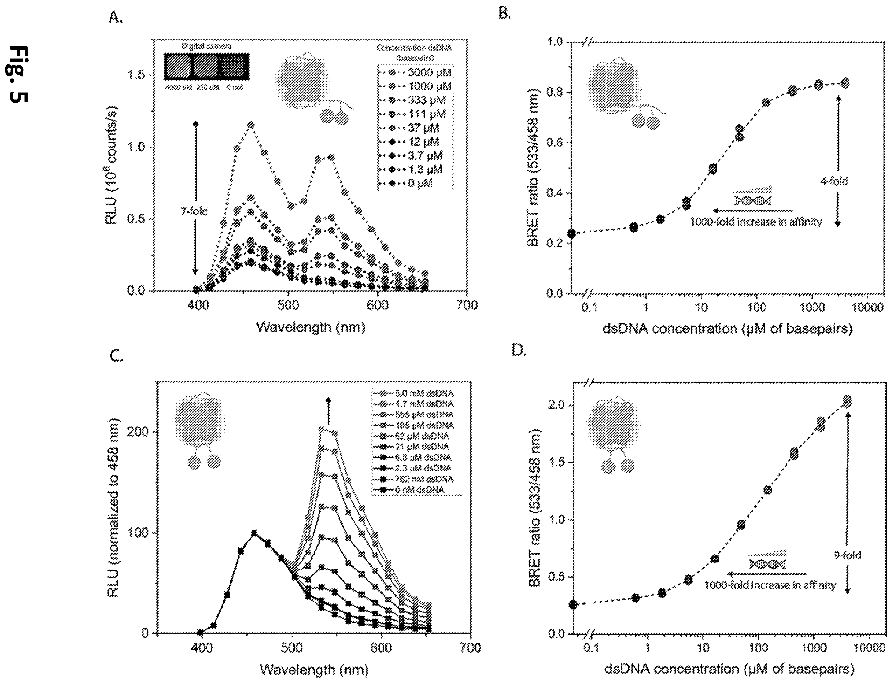

**Figure 5** | *(B,D) Bioluminescence titration of NanoLuc-2xTO with dsDNA with 2xTO located at the C-terminus (B) or in the flexible loop (D). Conjugates (1 nM) were added to a 3-fold dilution series* of *Salmon Sperm dsDNA ranging from 0.76 µM-5000 µM of basepairs. Incubation was performed at room temperature for 30 minutes in 1xPBS + 1 mg*/*mL BSA, 5% DMSO, pH 7.4. NanoGlo substrate (1000x dilution) was added, after which the emission intensity in the range of 398-653 nm was measured. Technical duplicates are represented as circles, and dashed lines connect mean values. (A, C) Full emission spectra of the bioluminescence titration of NanoLuc-2xTO with dsDNA with 2xTO located at the C-terminus (A) or in the flexible loop (C). The data points represent mean values. *Note that the data in panel B is identical to the data displayed in* *Figure 3BC* *(2 lysine linker, blue curve).*

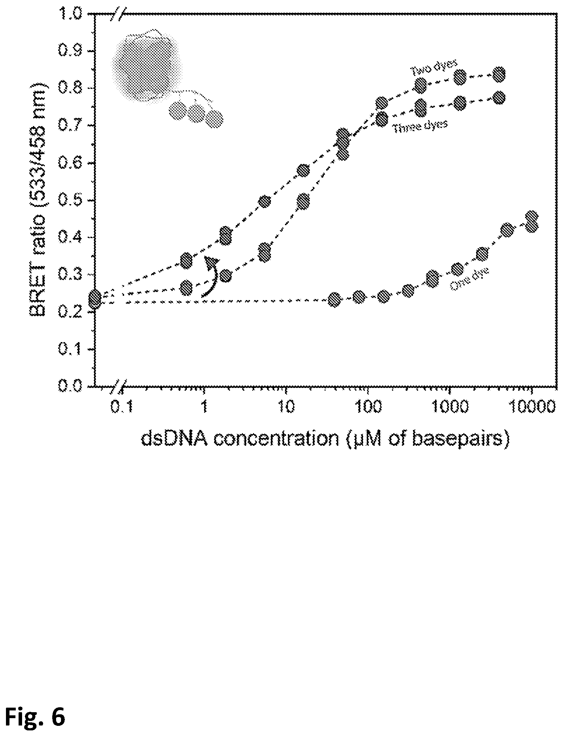

**Figure 6** **|** *Comparison of the bioluminescence titrations of NanoLuc-1xTO (green), NanoLuc-2xTO (blue) and NanoLuc-3xTO (red) with dsDNA, with TO located at the C-terminus. Conjugates (1 nM) were added to a 3-fold dilution series of Salmon Sperm dsDNA ranging from 0.76 µM-5000 µM of base pairs. Incubation was performed at room temperature for 30 minutes in 1xPBS + 1 mg*/*mL BSA, 5% DMSO, pH 7.4. NanoGlo substrate (1000x dilution) was added, after which the emission intensity in the range of 398-653 nm was measured. Technical duplicates are represented as circles, and dashed lines connect mean values.*
**Figure S2** | *A) schematic overview of the conjugation process, including the single cysteine NanoLuc variants, 1-(2-aminoethyl)maleimide crosslinker and the NHS-activated intercalating dye B) reaction scheme for coupling of the maleimide-activated crosslinker to NHS-TO C) LCMS results of NHS-TO before the coupling of the crosslinker (top) and after the coupling of the crosslinker (bottom). Molecular masses are indicated at the top of the chromatogram peaks.*

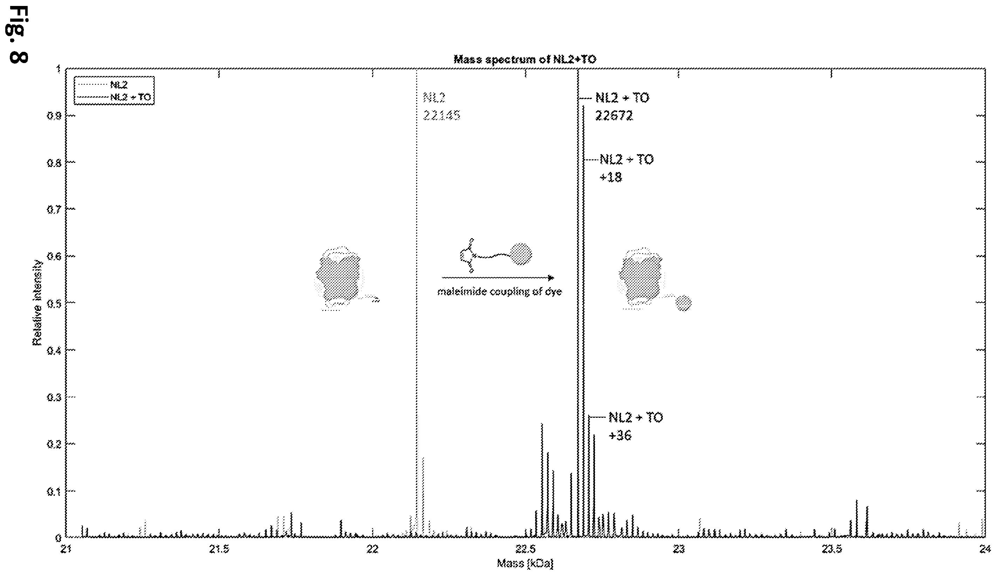

**Figure 8** | *Q-ToF LC-MS spectra of the NanoLuc variant with a single cysteine incorporated at the C-terminus, before (gray, NL2) and after (black, NL2 + TO) dye coupling. + 18 and + 36 peaks can be attributed to hydrolysis of the maleimide moiety and photobleaching of the dye. Calculated average molecular weight NL2 = 22145.3. Calculated average molecular weight NL2 + TO = 22672.2.*

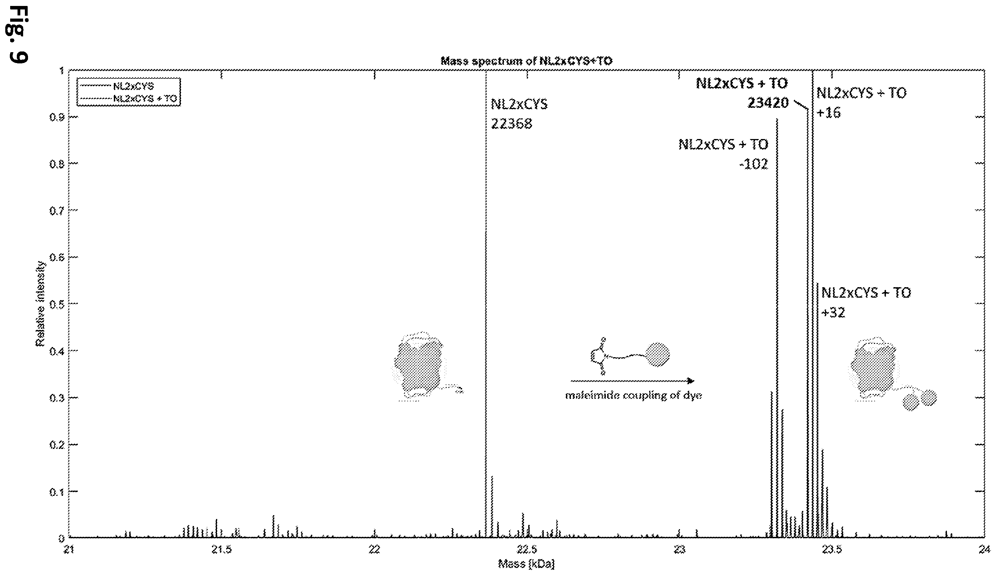

**Figure 9** **|** *Q-ToF LC-MS spectra of the NanoLuc variant with a two cysteines incorporated at the C-terminus, before (red, NL2xCYS) and after (black, NL2xCYS + TO) dye coupling. +18 and* + *36 peaks can be attributed to hydrolysis of the maleimide moiety and photobleaching of the dye. -102 peaks can be attributed to oxidative cleavage or side products as a results of conjugation of lysine residues with NHS-intercalating dye. Calculated average molecular weight NL2xCYS = 22367.6. Calculated average molecular weight NL2 +* TO = 23422.4.

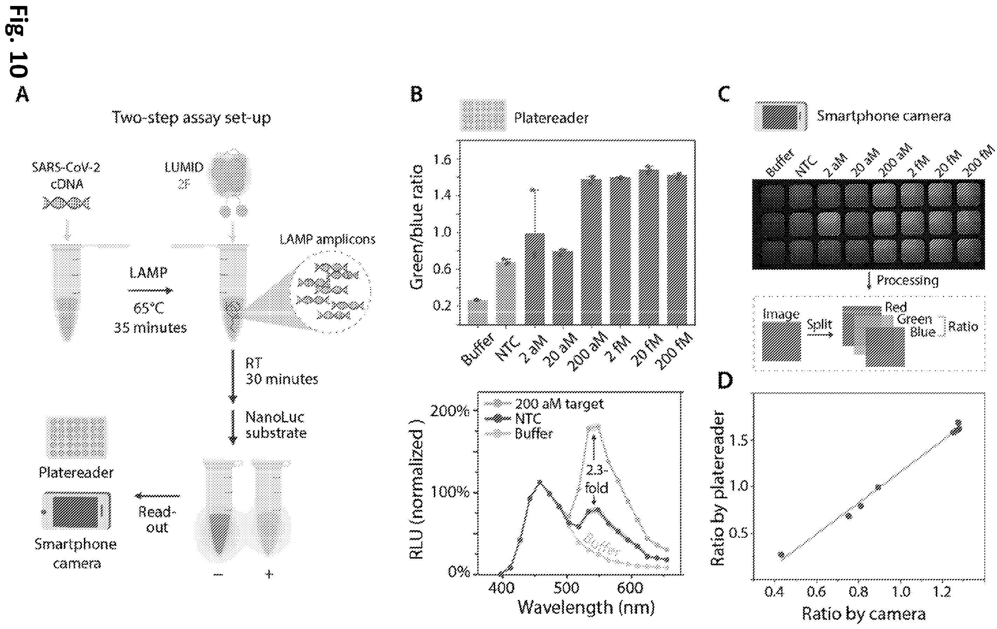

**Figure 10** **| Development of a two-step assay for the detection of SARS-CoV-2 cDNA. A** Schematic of the two-step assay set-up, in which SARS-CoV-2 cDNA is amplified using LAMP for 35 minutes at 65°C, then combined with 1 nM of LUMID-2F sensor and incubated for 30 minutes at room temperature before addition of Furimazine (1000x dilution) after which bioluminescence is measured using a plate reader and/or smartphone camera. **B** Sensor response of two-step assay, using a plate reader for detection. Data represents technical replicates, with n = 3 independent preparations of the cDNA. Top: green/blue ratio upon different input concentrations of cDNA, ranging from 2 aM (1.2 copies/µL) - 200 fM (120.000 copies/µL). Green/blue ratio was calculated by dividing the bioluminescent emission at 533 nm by emission at 458 nm, and represented as mean ± standard deviation with circles depicting individual data points. Bottom: full emission spectra displayed in buffer (grey), LAMP reaction without target cDNA (NTC, blue) and LAMP reaction with 200 aM of target cDNA (green). RLU (relative luminescence units) was normalized to the NanoLuc peak at 458 nm, and the spectra (bottom) are represented as mean ± standard deviation. C Sensor response of two-step assay, using a smartphone (Xiaomi mi 9 lite) inside a dark Styrofoam box. The photograph shows the sensor output for different input concentrations of cDNA, ranging from 2 aM (1.2 copies/µL) - 200 fM (120.000 copies/µL). Each column represents technical replicates, with n = 3 independent preparations of the cDNA. The same samples were used for detection with a smartphone camera (right) and the plate reader (left). **D** Correlation between the green/blue ratio calculated from the plate reader and smartphone camera.

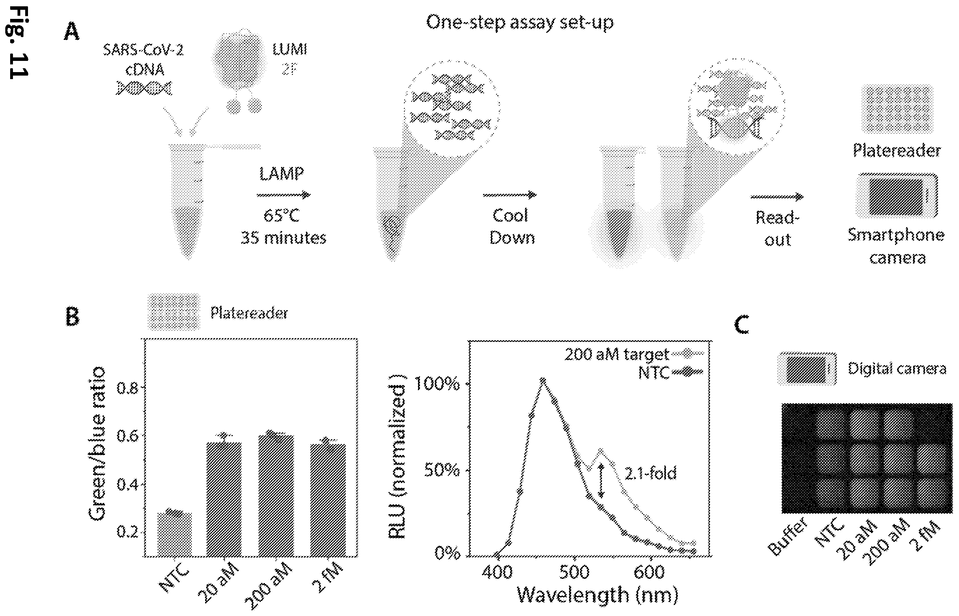

**Figure 11** **| Development of an one-pot assay for the detection of SARS-CoV-2 cDNA. A** Schematic of the one-pot assay set-up, in which all reaction components (SARS-CoV-2 cDNA, LUMID-2F sensor, NanoLuc substrate, and LAMP reaction components) are combined in a single-tube, and incubated for 35 minutes at 65°C. After cooling down, results are directly read out using a plate reader and/or smartphone camera. **B** Sensor response of the one-pot assay, using a plate reader for detection. Data represents technical replicates, with n = 3 independent preparations of the cDNA. Left: green/blue ratio upon different input concentrations of cDNA, ranging from 20 aM (12 copies/µL) - 2 fM (1.200 copies/µL). Green/blue ratio was calculated by dividing the bioluminescent emission at 533 nm by emission at 458 nm, and represented as mean ± standard deviation with circles depicting individual data points. Right: full emission spectra displayed in buffer (grey), LAMP reaction without target cDNA (NTC, blue) and LAMP reaction with 200 aM of target cDNA (green). Spectra (bottom) are represented as mean ± standard deviation. C Sensor response of single-step assay, using a conventional digital camera (Sony DSC-RX100) inside a dark Styrofoam box. The photograph shows the sensor output for different input concentrations of cDNA, ranging from 20 aM (12 copies/µL) - 2 fM (1.200 copies/µL). Each column represents technical replicates, with n = 3 independent preparations of the cDNA. The same samples were used for detection with a smartphone camera (right) and the plate reader (left).

### Summary of the invention

A readout based on bioluminescence circumvents the issue of requiring complex optical equipment based on lasers or LEDs, employing luciferase enzymes that produce light without requiring external excitation. We introduce a new class of bioluminescent sensor proteins based on a thermostable luciferase chemically connected to intercalating dyes that combine sensitive, non-specific detection of double-stranded DNA with a simple blue-to-green bioluminescent readout. Our invention thus makes it possible to perform quantitative, sensitive double-stranded DNA detection without any complex or highly specialized equipment, with a convenient optical readout that can be recorded with a simple digital camera or smartphone.

Therefore, in a first aspect is provided an intercalating dye probe for detection of double-stranded DNA, wherein the dye comprises a luminescence generating protein linked to one or more intercalating fluorescent dyes.

In a second aspect is provided a method of detecting double-stranded DNA in a solution, the method comprising: incubating the solution with the intercalating dye probe according to the first aspect of the invention and a suitable substrate for the luminescent domain; and detecting the light emitted by the intercalating fluorescent dye.

In a third aspect is provided a kit of parts comprising the intercalating dye probe according to the first aspect of the invention and a suitable substrate for the luminescent domain.

In a fourth aspect is provided the use of the intercalating dye probe according to the first aspect of the invention in a method of detecting double-stranded DNA.

### Detailed description of the invention

The present invention sought to develop a new class of bioluminescent sensor proteins that combine sensitive, non-specific detection of DNA with a simple camera-based readout that is suitable for point-of-care diagnostics. To this end, we chemically conjugated one or more intercalating dyes to the thermostable NanoLuc luciferase, which emits blue-light bioluminescence upon the addition of a substrate. In absence of dsDNA, the intercalating dyes will be minimally fluorescent and only the blue light of NanoLuc is witnessed. In presence of dsDNA, the intercalating dyes bind to the dsDNA, enabling bioluminescent resonance energy transfer (BRET) from NanoLuc to the dye, after which green light is emitted (Figure 1). The ratio between the emission of NanoLuc and intercalating dye then provides a measure for the amount of dsDNA in the sample. We aim to combine these probes with isothermal amplification strategies such as RPA and LAMP to provide a pre-amplification step in order to further increase assay sensitivity. This approach thus allows simple and sensitive detection of DNA without the use of high-tech equipment and an external excitation source, opening up possibilities for application in a point-of-care setting where access to resources or trained personnel might be limited.

Herein provided is thus a simple method for the detection of DNA without the need for specialized personnel or equipment, which can be applied in diagnosis and prevention of infectious diseases. The invention describes a novel molecular probe that consists of two parts: a light-emitting bioluminescent protein, which is directly attached to an intercalating dye. When the probe binds to DNA both parts interact causing the light that is produced to change color, e.g. from blue to green. The probe is simply added to a sample, after which the color change indicating the presence of DNA can be recorded and quantified using a simple camera or smartphone.

Therefore, in a first aspect is provided an intercalating dye probe for detection of double stranded DNA, wherein the dye comprises a luminescence generating protein linked to one or more intercalating fluorescent dyes. The luminescence generating protein may be a bioluminescent protein or an enzyme used for enhancing chemiluminescence.

When used herein, the term luciferase refers to a protein capable of creating bioluminescence in the presence of an appropriate substrate. Such protein may be a naturally occurring protein (e.g. a protein isolated from an organism exhibiting bioluminescence) or a modification or synthetic variant thereof. It is understood that the term luciferase refers to a broad variety of unrelated proteins which have in common the ability to exhibit bioluminescence (emitting light in the presence of a substrate), therefore any protein able to exhibit bioluminescence is for the purpose of the invention deemed a bioluminescent protein. When used herein the term luciferase activity refers to the ability of a protein to convert a substrate resulting in light emission (bioluminescence). Therefore, in an embodiment, the bioluminescent protein is a protein with luciferase activity, preferably selected from: nanoluc, firefly luciferase, renilla luciferase, metridia luciferase, bacterial luciferase, dinoflagellate luciferase, oplophorus luciferase, Gaussia luciferase, TurboLuc luciferase, Aluc luciferase, or catalytically active fragments thereof.

The bioluminescent protein is directly linked to the intercalating dye probe, enabling transfer of the emitted light from the bioluminescent protein to the intercalating dye, resulting in excitation and subsequent fluorescence of the dye. As the dye will only display fluorescence when intercalated in double-stranded DNA, the emission of the dye specific can be used to detect or quantify double-stranded DNA (dsDNA) as described herein below.

When used herein the term "linked" refers to a covalent bond. Methods of linking a dye to a protein are commonly known in the field. For example, the dye may be linked to a cysteine residue in the protein as described below. A cysteine may be introduced in the protein, e.g. when no suitable cysteine is available, by point mutation. It is further understood that multiple cysteines may be introduced to allow linking to multiple dyes. Preferably the cysteines are spatially separated, either by introducing them in different regions of the protein or by including a linker sequence, for example a 1 Lysine linker, a 2 Lysine linker or a 3 Lysine linker. Further, cysteines present in the protein at unsuitable locations may be removed by point mutation. Therefore, in an embodiment the protein with luciferase activity comprises an amino acid sequence as defined by any one of SEQ ID Nos 1 to 7.
overview of sequences use in this application:

| SEQ ID NO: | description: |
|---|---|
| 1 | Nanoluc with N-terminal STREP tag and C-terminal His tag and C166S point mutation, with G182C mutation for C-terminal linkage of a single dye. |
| 2 | Nanoluc with N-terminal STREP tag and C-terminal His tag and C166S point mutation, with G182CKC mutation for C-terminal linkage of two dyes with single lysine linker. |
| 3 | Nanoluc with N-terminal STREP tag and C-terminal His tag and C166S point mutation, with G182CKKC mutation for C-terminal linkage of two dyes with double lysine linker. |
| 4 | Nanoluc with N-terminal STREP tag and C-terminal His tag and C166S point mutation, with G182CKKKC mutation for C-terminal linkage of two dyes with triple lysine linker. |
| 5 | Nanoluc with N-terminal STREP tag and C-terminal His tag and C166S point mutation, with G182CKKCKKC mutation for C-terminal linkage of three dyes with two double lysine linkers. |
| 6 | Nanoluc with N-terminal STREP tag and C-terminal His tag and C166S point mutation, with D148C mutation for flexible loop linkage of a single dye. |
| 7 | Nanoluc with N-terminal STREP tag and C-terminal His tag and C166S point mutation, with D148CKKC mutation for flexible loop linkage of two dyes with a double lysine linker. |

The above protein sequences comprise:
- start codon (Met, 1^{st} amino acid);
- Streptavidin (STREP) tag - (Trp-Ser-His-Pro-Gln-Phe-Glu-Lys; amino acids 2 to 9);
- modified NanoLuc protein sequence (starting at amino acid 10); and
- 6 Histidine tag (Last 6 amino acids).

The numbering for the mutations in the above sequences follows the native nanoluc sequence, thus excluding the STREP-tag and starting at the methionine at position 10 of the presented sequences.

Alternatively, the dye can be coupled to an enzyme which produces a substrate enabling chemiluminescence. For example a horseradish peroxidase may be coupled to allow catalysis of the oxidation of luminol using hydrogen peroxide. Therefore, in an embodiment the enzyme used for enhancing chemiluminescence is selected from horse radish peroxidase and alkaline phosphatase.

Suitable intercalating fluorescent dyes are known to the skilled person. In an embodiment the intercalating fluorescent dye is selected from acridine orange, thiazole orange, ethidium bromide, SYBR green I, SYBR Gold, SYBR Safe, EvaGreen, EvaRuby, PicoGreen, SYTO-9, TOTO-1, and YOYO-1. It is understood that the intercalating dye probe may comprise one or more, such as one, two, three, four, five or more intercalating fluorescent dyes. Multiple fluorescent intercalating dyes may be added to enhance DNA targeting of the probe. In an embodiment the intercalating dye probe further comprises an additional DNA-binding domain. This may advantageous to further enhancing binding or targeting of DNA.

In a second aspect the invention relates to a method of detecting double-stranded DNA in a solution, the method comprising:
- incubating the solution with the intercalating dye probe according to the first aspect and a suitable substrate for the luminescent domain; and
- detecting the light emitted by the intercalating fluorescent dye.

As the intercalating dye can only be excited by the luminescence generating protein when intercalated in double-stranded DNA, detecting light emitted by the dye can be used to detect dsDNA. By also detecting the light emitted due to the luminescence generating protein (e.g. directly by a bioluminescent protein or indirectly by an enzyme for enhancing chemiluminescence), the amount of dsDNA can be quantified. Therefore, in an embodiment the method is used to quantify double-stranded DNA, the method further comprising:
- detecting the light emitted by the luminescent domain; and
- quantifying the amount of double stranded DNA based on the ratio of the light emitted by the intercalating fluorescent dye and the light emitted by the luminescent domain. The method may be performed before, during and/or after a DNA amplification reaction. The method may be performed once or continuously during an amplification reaction.

It is understood that the method may be performed in any type of amplification reaction for amplifying dsDNA. In an embodiment the amplification reaction is selected from: polymerase chain reaction (PCR), recombinase polymerase amplification (RPA), loop mediated isothermal amplification (LAMP), nucleic acid sequence based amplification (NASBA), strand displacement amplification (SDA), multiple displacement amplification (MDA), rolling circle amplification (RCA), ligase chain reaction (LCR), helicase dependent amplification (HDA) or ramification amplification method (RAM). In a third aspect the invention relates to a kit of parts comprising the intercalating dye probe according to the first aspect of the invention and a suitable substrate for the luminescent domain. In an embodiment the substrate is luciferin, preferably furimazine, firefly luciferin, latia (snail) luciferin, bacterial luciferin, coelenterazine, dinaflagellate luciferin, vargulin, 3-hydroxy hispidin or luminol. It is understood that luciferin is the general term for a compound that acts as the substrate for a luciferase, therefore the term luciferin when used herein refers to any suitable substrate of a luciferase as defined herein.

In a fourth aspect the invention relates to the use of the intercalating dye probe according to the first aspect of the invention in a method of detecting double-stranded DNA.

Other variations to the disclosed realizations can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

### Examples

### Materials and methods

### Cloning

The pET28a vector containing DNA encoding the NanoLuc luciferase with an N-terminal Strep-tag and C-terminal hexahistidine-tag was ordered from GenScript. Sitedirected mutagenesis to mutate the native cysteine to a serine (C166S) and introduce new cysteine and lysine residues was carried using the QuikChange Lightning Site-Directed Mutagenesis kit (Agilent) using specific primers according to the manufacturer's instructions. All cloning and mutagenesis results were confirmed by Sanger sequencing (BaseClear).

### Protein expression and purification

The plasmids encoding NanoLuc were transformed into chemically competent *E. coli* BL21 (DE3) and cultured in 2YT medium (16 g peptone, 5 g NaCl, 10 g yeast extract per liter) supplemented with 50 µg/mL kanamycin. At OD₆₀₀ = 0.6, protein expression was induced using 1 mM isopropyl β-D-1-thiogalactopyramoside (IPTG) overnight at 20°C. Subsequently, cells were harvested by centrifugation and lysed using Bugbuster protein extraction reagent (Novagen), supplemented with Benzonase endonuclease (Novagen). Proteins were purified using Ni²⁺-NTA affinity chromatography, after which the elution fractions were exchanged to storage buffer (100 mM Tris-HCl, 150 mM NaCl, pH 8.0). Protein purity and correct mass were confirmed by SDS-PAGE and Q-ToF LC-MS, respectively. Purified proteins were stored at -80°C until conjugation.

### Conjugation of NHS-activated dye to 1-(2-aminoethyl)maleimide crosslinker

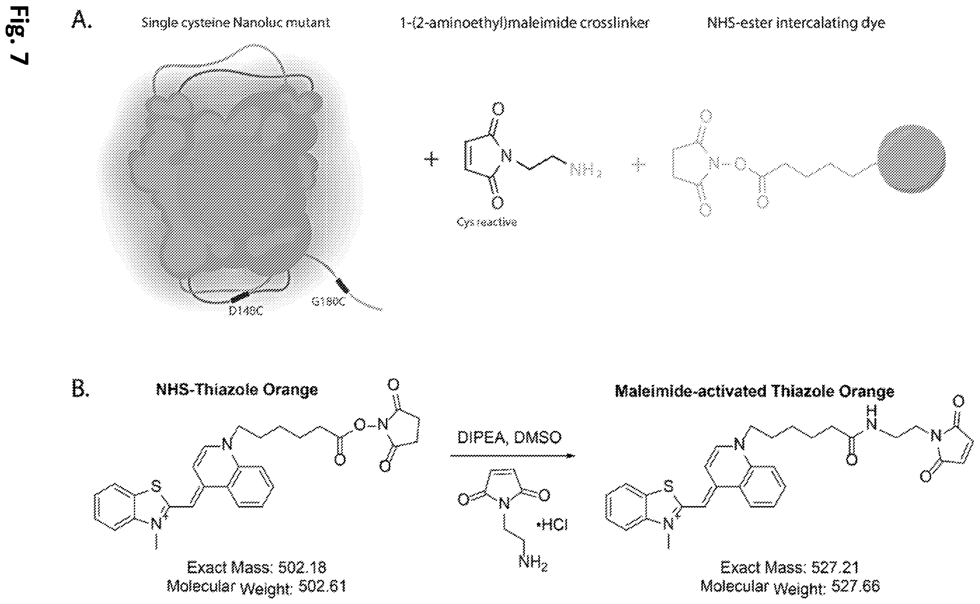

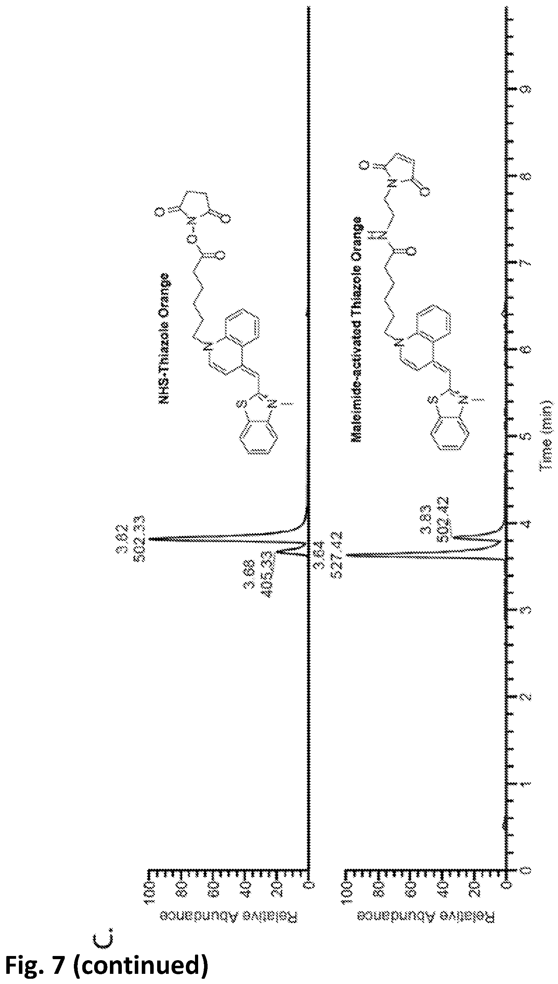

The amine-reactive N-hydroxysuccinimide (NHS) esters of Thiazole Orange (TO) and Acridine Orange (AO) were obtained from Biotium and each dissolved in DMSO to a final concentration of 20 mM. Subsequently, 3 equivalents of N,N-diisopropylethylamine (DIPEA) and 0.8 equivalents of 1-(2-aminoethyl)maleimide crosslinker (Sigma Aldrich) were added and incubated overnight at room temperature with continuous shaking at 450 rpm (Figure 7B). The correct mass after conjugation was confirmed using LC-MS (Figure 7C). The maleimide-activated dyes were stored at -30°C until further use.

### Conjugation of maleimide-activated dyes to NanoLuc

Before conjugating NanoLuc to the maleimide-activated dyes, the protein was first reduced by incubating with 5 mM of TCEP for 1 hour at room temperature with continuous shaking at 500 rpm and subsequently buffer exchanged to a sodium phosphate buffer (100 mM NaPi, 25 µM TCEP, pH 7.0) using a PD-10 desalting column (GE Healthcare). Then, the maleimide-activated TO and AO were both added in a 10-fold molar excess to 10 µM of reduced NanoLuc and allowed to react for 2 hours at room temperature with continuous shaking at 500 rpm. The NanoLuc-dye conjugates were purified by a PD-10 desalting column to remove excess dye and simultaneously buffer exchanged to PBS (100 mM NaPi, 150 mM NaCl, pH7.2). The coupling and purification were checked using SDS-PAGE by exciting the dye-protein conjugates with 470-nm blue light. Finally, the coupling efficiency and correct mass of the NanoLuc-dye conjugates were confirmed by Q-ToF LC-MS.

### Bioluminescent assays

Bioluminescent assays were performed at sensor protein concentrations of 1 nM in a total volume of 20 µL in PerkinElmer flat white 384-well Optiplate. Sheared Salmon Sperm dsDNA fragments of ~ 2000 bp were ordered from Thermo Fisher and diluted to a concentration range of 0.7 µM - 5 mM, measured in terms of the number of base pairs. After incubation of sensor proteins and dsDNA fragments for 0.5 hours at room temperature, NanoGlo substrate (Promega, N1110) was added at a final dilution of 1:1000. Bioluminescence spectra were recorded in a plate reader (Tecan Spark 10M), recorded full spectra between 398 nm and 653 nm with a step size of 15 nm, a bandwidth of 25 nm and an integration time of 250 ms.

### Two-step SARS-CoV-2 cDNA assays.

LAMP primers targeting the cDNA sequence of the Nucleocapsid (N)-gene of the SARS-CoV-2 virus were designed using the NEB LAMP Primer Design Tool and ordered from IDT. The sequence of the N-gene can be found in SEQ ID NO: 8. Primers were diluted to a 10 x concentrated stock, containing 16 µM of inner primers, 2 µM of outer primers and 4 µM of loop primers. A plasmid containing SARS-CoV-2 cDNA sequences was obtained through the Free Genes Project and using specific primers, the nucleocapsid (N)-gene sequence was PCR amplified from this plasmid. The N-gene target DNA was serially diluted to 25 x concentrated stocks ranging from 5 aM - 5 pM. Positive LAMP reactions were assembled in a UV PCR cabinet by combining 1 x isothermal amplification buffer (NEB), 6 mM of MgSO₄ (NEB), 1.4 mM of dNTPs (NEB), 1 x LAMP primer mix and 1 x target DNA in a total volume of 24 µL. For the non-template control, similar conditions were used, only interchanging the target DNA for MilliQ water. Reactions were kept on ice during the full assembly process. To initiate the reactions, Bst 2.0 polymerase (8 U, NEB) was added followed by incubation at 65°C for 35 minutes. Next, 10 µL of each LAMP reaction was combined with 10 µL of LUMID-2F sensor (2 nM in PBS buffer (pH 7.4, 0.1% (w/v) BSA, 5% DMSO)) in a PerkinElmer flat white 384-well Optiplate. For a negative control, the LAMP reaction was substituted for PBS buffer. After incubation for 30 minutes at room temperature, NanoGlo substrate (Promega, N1110) was added at a final dilution of 1:1000. Luminescence spectra were recorded in a plate reader (Tecan Spark 10M) between 398 nm and 653 nm with a step size of 15 nm, a bandwidth of 25 nm and an integration time of 100 ms. The green/blue ratio was calculated by dividing bioluminescent emission at 533 nm by emission at 458 nm. The luminescence signal was also recorded using a smartphone (Xiaomi mi 9 lite) camera through a hole in a Styrofoam box to exclude the surrounding light. Photographs were taken with an exposure time of 32 s, and ISO value of 3200.

### One-pot SARS-CoV-2 cDNA assays.

LAMP reaction were assembled as described in the two-step assay, but with the addition of 1 µL of LUMID-2F sensor (25 nM in PBS buffer (pH 7.4, 0.1% (w/v) BSA, 5% DMSO)) and 1 µL of NanoGlo substrate (1:40 dilution). To initiate the reactions, Bst 2.0 polymerase (8 U, NEB) was added followed by incubation at 65°C for 35 minutes. Real-time Luminescence was monitored using a digital camera (Sony DSC-RX100) through a hole in a dark Styrofoam box containing a heating plate to maintain the reaction temperature. After incubation, reactions were transferred to room temperature and allowed to cool down for 5 minutes before recording the Luminescence signal. Luminescence spectra were recorded in a plate reader (Tecan Spark 10M) between 398 nm and 653 nm with a step size of 15 nm, a bandwidth of 25 nm and an integration time of 100 ms. The green/blue ratio was calculated by dividing bioluminescent emission at 533 nm by emission at 458 nm. The luminescence signal was also recorded using the set up for the real-time Luminescence monitoring. Photographs were taken with an exposure time of 30 s, and ISO value of 6400.

### Results and discussion

### Single-dye NanoLuc variants

The synthesis of the luciferase-intercalating dye probes was performed by coupling single-cysteine NanoLuc variants through a hetero-bifunctional 1-(2-aminoethyl)maleimide crosslinker to the NHS-activated form of the intercalating dyes Thiazole Orange (TO) or Acridine Orange (AO). TO was selected based on its large increase in fluorescence (> 3000-fold) upon complexation with dsDNA, appropriate spectral overlap with NanoLuc (em. Nanoluc 460 nm, ex. TO 514 nm), and the commercial availability of an NHS-activated form. AO is an alternative intercalating dye that has been used as a probe to discriminate between double-stranded and single-stranded nucleic acids. Although AO is already fluorescent in solution and has a low intrinsic increase in fluorescence upon dsDNA binding, its strong intrinsic fluorescence was exploited to analyze the conjugation procedure. To allow for sitespecific conjugation, single cysteine mutations were incorporated in the flexible loop region (D148) and C-terminus (G182) of NanoLuc, which are known to be suitable positions for conjugation without interfering with NanoLuc's bioluminescent properties. A hexahistidine-tag at the C-terminus was included to facilitate the purification of the single-cysteine NanoLuc mutants (the DNA and amino acid sequences and a list of protein mutants can be found in the Table above). The 1-(2-aminoethyl)maleimide crosslinker was first conjugated to the NHS-activated intercalating dyes, after which the reaction product was coupled to NanoLuc (**Figure 3****,** **Figure 7**). Q-ToF LC-MS analysis revealed that the synthesis was successful, although minor peaks attributed to photobleaching and hydrolysis of the maleimide functionality were observed (Figure 8).

In order to test the analytical performance of NanoLuc-Thiazole Orange probes towards dsDNA, bioluminescence titrations with dsDNA were performed (Figure 2). The results reveal that both probes were able to intercalate into the dsDNA, judging by the increase in the emission of green light (~ 533 nm) upon increasing the amount of dsDNA (**Figure 2AB,** middle). Also, the ratio between the emission of Thiazole Orange (533 nm) and the emission of NanoLuc (458 nm), or the 'BRET ratio', increased with increasing concentrations of dsDNA, further confirming the dsDNA dependency of the sensor (**Figure 2AB,** right). Nevertheless, BRET only appeared at high concentrations of dsDNA (~ mM range), indicating that the affinity of the probe for dsDNA is low. The increase in BRET ratio upon addition of dsDNA was bigger for the construct with the dye positioned in the flexible loop region (**Figure 2A****,** 4 fold) compared to the construct with the dye position at the C-terminus (**Figure 2B****,** <2 fold). Since BRET is strongly distance dependent, we can attribute this difference to the distance between the dye and the active site of NanoLuc in both mutants, i.e. positioning of the dye at the flexible C-terminus (G182C) leads to a larger distance to the active site compared to the D148C mutant, and therefore a reduced BRET efficiency.

### Multiple-dye NanoLuc variants - linker optimization

A promising engineering strategy to increase the overall affinity of the probes is to introduce multivalency within the current system by incorporating multiple dyes. The development of a dimeric Thiazole Orange dye (TOTO) has been shown to increase the affinity to dsDNA about three orders of magnitude compared to the monomeric variant¹³. Similarly, small peptides containing two Acridine Orange dyes separated by two lysine residues that provide additional, electrostatic interactions with the dsDNA, were shown to increase the dye's affinity by two to three orders of magnitude¹⁴. To apply the concept of multiple dyes with a positively charged linker to our probes, a second intercalating dye was incorporated in close proximity to the first intercalating dye at the C-terminus of Nanoluc (C180), separated by lysine residues. The amount of lysines was varied from one to three in order to find the optimal distance the dyes to bind and intercalate into the dsDNA, and glycine residues were incorporated as a control to confirm the necessity of the positive charges (**Figure 3A**). Bioluminescence titration with dsDNA revealed that the double-lysine linker responded with the highest affinity and highest dynamic range to the dsDNA (**Figure 3B****,** blue). The variant with the triple-lysine linker was found to have a comparable affinity, but with a smaller dynamic range that can be attributed to the dyes not being able to both effectively intercalate into the dsDNA, resulting into less green fluorescence (**Figure 3B****,** red). The lowest affinity and dynamic range was observed for the single-lysine linker, which can be explained by the combination of a the smaller amount of positive charges and non-optimal distance for the dyes to both bind the dsDNA (**Figure 3B****,** green). The control experiment with the 2 glycine linker showed a ~100x lower affinity compared to the 2 lysine linker, showing that the positive charges are essential for improved dsDNA binding (**Figure 3C**) **.**

### Multiple-dye NanoLuc variants - 2 dyes

Next, 2 cysteines separated by the best-performing 2-lysine linker were incorporated at the C-terminus and flexible loop region of NanoLuc (NanoLuc-2xTO, **Figure 4**). Q-ToF LC-MS analysis revealed that the coupling reaction was complete, although peaks attributed to photobleaching and hydrolysis of the maleimide functionality were observed (Figure 9).

In order to test the analytical performance of the NanoLuc-2xTO sensors towards dsDNA, again bioluminescence titrations with dsDNA were performed (**Figure 5**). Upon the addition of increasing concentrations of dsDNA, an increase in the ratio between the emission of Thiazole Orange (533 nm) and the emission of NanoLuc (458 nm) was observed (BRET ratio). BRET already appeared within the low-µM range, indicating a 1000-fold increase in affinity compared to the sensor variant containing only a single TO (compare with **Figure 2**). A moderate 4-fold increase in BRET ratio was observed for the variant carrying 2xTO at the C-terminus, while a 9-fold increase was observed for the variant with 2xTO in the flexible loop (compare **Figure 5B** and D). Similar to the results obtained for the single-dye variants in **Figure 2****,** these results illustrate that BRET efficiency is strongly distance dependent and that positioning of the intercalating dyes in the flexible loop increases BRET efficiency.

Interestingly, the full emission spectra reveal a positive correlation between the total emission intensity and the amount of dsDNA, which could also been seen by differences in brightness in a digital camera picture (**Figure 5A**). It is hypothesized that the addition of a second dye increases the hydrophobicity of the system, and thereby decreases the solubility of NanoLuc-2xTO, in turn decreasing the signal strength. Large amounts of negatively charged dsDNA might act to stabilize the protein-dye construct or prevent the formation of aggregates, leading to the observed increase in absolute signal.

### Multiple-dye NanoLuc variants - 3 dyes

We also assessed the sensor performance when a third dye, separated by a 2-lysine linker, was added to the C-terminus of NanoLuc (NanoLuc-3xTO, **Figure 6**). From titrations with dsDNA, a lower limit of detection (LoD) and higher affinity was observed compared to the variant with two dyes. Additionally, the curve seems to be stretched over a larger range of dsDNA concentrations with a smaller fold change in BRET ratio. This may be an indication that a mixture of different affinity binders was present, which can be ascribed to an incomplete coupling reaction of the dyes. Consequently, this results in NanoLuc variants with either one, two or three dyes that bind the dsDNA in a different concentration regime.

The present invention describes a generic bioluminescent sensor for dsDNA detection based on the coupling of one or more intercalating dyes to a light-emitting luciferase protein. Combining multivalent DNA binding of more than one dye per probe and the use of short positively charged lysine linkers between dyes resulted in high-affinity DNA detection probes with a simple bioluminescent readout. Results show that dsDNA could be detected with a ~µM affinity (measured in base pairs) and a 4 to 9-fold change in BRET ratio. In context, most amplification strategies generate dsDNA fragments of 100-200 bp, which suggest that the sensors developed here are already capable of detecting such fragments in the low-nM range.

The present invention is envisioned to be used for the detection of viral DNA or RNA in patient samples such as blood and saliva, using rhinoviruses causing the common cold or the highly relevant and disruptive SARS-CoV-2 virus. To this end, the herein described bioluminescent intercalating proteins could be combined with amplification techniques (e.g. RPA or LAMP), which provide a straightforward method to perform an initial pre-amplification step in order to increase the sensitivity of the assay required for the detection of DNA directly in patient samples. Because of the thermal stability of NanoLuc, we expect that the NanoLuc-TO conjugates can be used to monitor the progression of RPA (T = 42 °C) in a one-pot reaction in real-time. It is further envisioned that the present invention further increases the affinity of the luciferase-TO conjugates for dsDNA by combining bivalent TO labeling at the loop position with bivalent labeling at the C-terminus. Further envisioned are fusions of the NanoLuc domain to a generic dsDNA binding domain to further increase sensitivity.

### Two-step assay for the detection of SARS-CoV-2 complementary DNA

In context, dsDNA fragments are typically 100-200 base pairs long, suggesting that the sensors developed here are capable of detecting such fragments in the lownanomolar range. Since most diagnostic applications, such as viral nucleic acid detection, require attomolar sensitivity, we next explored combining our bioluminescent probes with different isothermal amplification steps to develop a simple and sensitive assay platform that can be used at the point of care. Of the methods explored, LAMP was found most suitable due to the high dsDNA yield with minimal non-specific amplification, which is essential to avoid large background signals when employing a non-specific read-out. Motivated by the ongoing COVID-19 pandemic, LAMP reactions were designed to target the complementary DNA (cDNA) sequence of the nucleocapsid (N)-gene of the SARS-CoV-2 virus, exploring the feasibility of the LUMID probes for a rapid, point-of-care test for viral detection.

We first developed a two-step assay in which serially diluted SARS-CoV-2 cDNA was amplified using LAMP and subsequently combined with LUMID-2F to facilitate bioluminescent detection (**Figure 10A**). To this end, LAMP reactions were performed according to manufacturer's instructions for 35 minutes at 65°C, and then 1:1 (v/v) combined with 2 nM of sensor protein. Following 30 minutes of incubation and the addition of substrate, DNA concentrations down to 200 aM (120 copies/µL) could be discriminated from the non-template control (NTC) with a 2.5-fold change in green/blue emission ratio (**Figure 10B**). To illustrate the potential for point-of-care applications, we also recorded the signal using a standard smartphone camera (Xiaomi mi 9 lite) in a dark Styrofoam box. Photographs taken of the same samples as used for the plate reader measurements showed a clear visual color change from blue to green for all DNA concentrations down to 200 aM (**Figure 10C**). The exact emission ratios were calculated from the blue- and green-color channels of the RGB image of the smartphone and showed a linear correlation with the ratio obtained from the plate reader measurements (**Figure 10D**), with a Pearson's coefficient of 0.996. This reveals that while the absolute changes in emission ratio are different, the performance of the smartphone camera is comparable to that of the plate reader. For both detection methods, the amount of green light in the NTC was found higher than in the control with only buffer, which can be ascribed to the long (~40 bp) primers used in the LAMP reaction that form detectable secondary dsDNA structures without amplification. Although this primer-related background signal decreases the assay's dynamic range, the observed change in emission ratio is robust and comparable to BRET-based sensor's in general. These results illustrate that our LUMID sensors can be combined with LAMP to create a sensitive nucleic acid assay platform in which results can be obtained in approximately 1 hour using simple, smartphone-based detection.

### One-pot assay for the detection of SARS-CoV-2 complementary DNA

We next assessed whether we could combine all assay components in a single tube, demonstrating the viability of this platform as a single-step point-of-care diagnostic tool. This approach would not only simplify the experimental procedure, but also reduce the risk at false-positives that may arise as a result of cross-contamination during post-amplification reaction transfer. We therefore combined the LAMP reaction components with serially diluted SARS-CoV-2 cDNA, LUMID-2F sensor (1 nM) and NanoLuc substrate (1000x dilution) in a single tube, before incubation for 35 minutes at 65°C (**Figure 11A**). Although NanoLuc was found inactive at the reaction temperature of 65°C, a 5-minute cool-down at room temperature was sufficient to fully recover its enzymatic activity. Using this short cool-down step, DNA concentrations down to 20 aM (12 copies/µL) could be discriminated from the non-template control (NTC) with a 2.1-fold change in green/blue emission ratio (**Figure 11B**). Also in photographs taken of the same samples as used for the plate reader measurements, a visual color change could be seen for DNA concentrations down to 20 aM (**Figure 11C**). This indicates that translation towards a one-pot assay did not compromise on sensitivity, maintaining the attomolar sensitivity as reported for the two-step assay. Although an apparent 10-fold increase in affinity was observed, this is likely due to reaction-to-reaction variability intrinsic to the LAMP reaction.

## Claims

1. An intercalating dye probe for detection of double stranded DNA, wherein the dye comprises a luminescence generating protein linked to one or more intercalating fluorescent dyes.

2. The intercalating dye probe according to claim 1, wherein the luminescence generating protein is a bioluminescent protein or an enzyme used for enhancing chemiluminescence.

3. The intercalating dye probe according to claim 2, wherein the bioluminescent protein is a protein with luciferase activity, preferably selected from: nanoluc, firefly luciferase, renilla luciferase, metridia luciferase, bacterial luciferase, dinoflagellate luciferase, oplophorus luciferase, Gaussia luciferase, TurboLuc luciferase, Aluc luciferase, or catalytically active fragments thereof.

4. The intercalating dye probe according to claim 3, wherein the protein with luciferase activity comprises an amino acid sequence as defined by any one of SEQ ID Nos 1 to 7.

5. The intercalating dye probe according to claim 2, wherein the enzyme used for enhancing chemiluminescence is selected from horse radish peroxidase and alkaline phosphatase.

6. The intercalating dye probe according to any one of the previous claims, wherein the intercalating fluorescent dye is selected from acridine orange, thiazole orange, ethidium bromide, SYBR green I, SYBR Gold, SYBR Safe, EvaGreen, EvaRuby, PicoGreen, SYTO-9, TOTO-1, and YOYO-1.

7. The intercalating dye probe according to any one of the previous claims, wherein the intercalating dye probe further comprises an additional DNA binding domain.

8. A method of detecting double stranded DNA in a solution, the method comprising:
- incubating the solution with the intercalating dye probe according to any one of claims 1 to 7 and a suitable substrate for the luminescent domain; and
- detecting the light emitted by the intercalating fluorescent dye.

9. The method according to claim 8, wherein the method is used to quantify double stranded DNA, the method further comprising:
- detecting the light emitted by the luminescent domain; and
- quantifying the amount of double stranded DNA based on the ratio of the light emitted by the intercalating fluorescent dye and the light emitted by the luminescent domain.

10. The method according to claim 8 or 9, wherein the method is performed before, during and/or after a DNA amplification reaction.

11. The method according to claim 10, wherein the amplification reaction is selected from: polymerase chain reaction (PCR), recombinase polymerase amplification (RPA), loop mediated isothermal amplification (LAMP), nucleic acid sequence based amplification (NASBA), strand displacement amplification (SDA), multiple displacement amplification (MDA), rolling circle amplification (RCA), ligase chain reaction (LCR), helicase dependent amplification (HDA) or ramification amplification method (RAM).

12. A kit of parts comprising the intercalating dye probe according to any one of claims 1 to 7 and a suitable substrate for the luminescent domain.

13. Kit of parts according to claim 12, wherein the substrate is luciferin, preferably furimazine, firefly luciferin, latia (snail) luciferin, bacterial luciferin, coelentrazine, dinaflagellate luciferin, vargulin, 3-hydroxy hispidin, luminol or H₂O₂.

14. Use of the intercalating dye probe according to any one of claims 1 to 7 in a method of detecting double stranded DNA.

## Patentansprüche

1. Interkalationsfarbstoff-Sonde zum Nachweis doppelsträngiger DNA, wobei der Farbstoff ein Lumineszenz-erzeugendes Protein umfasst, das mit einem oder mehreren Fluoreszenz-Interkalationsfarbstoffen verknüpft ist.

2. Interkalationsfarbstoff-Sonde nach Anspruch 1, wobei es sich bei dem Lumineszenz-erzeugenden Protein um ein Biolumineszenzprotein oder ein zum Verstärken von Chemolumineszenz verwendetes Enzym handelt.

3. Interkalationsfarbstoff-Sonde nach Anspruch 2, wobei es sich bei dem Biolumineszenzprotein um ein Protein mit Luciferase-Aktivität handelt, das vorzugsweise aus Folgendem ausgewählt wurde: Nanoluc, Firefly-Luciferase, Renilla-Luciferase, Metridia-Luciferase, Bakterien-Luciferase, Dinoflagellat-Luciferase, Oplophorus-Luciferase, Gaussia-Luciferase, TurboLuc-Luciferase, Aluc-Luciferase oder katalytisch aktiven Fragmenten davon.

4. Interkalationsfarbstoff-Sonde nach Anspruch 3, wobei das Protein mit Luciferase-Aktivität eine wie in einer aus SEQ ID Nr. 1 bis 7 definierte Aminosäuresequenz umfasst.

5. Interkalationsfarbstoff-Sonde nach Anspruch 2, wobei das zum Verstärken von Chemolumineszenz verwendete Protein aus Meerrettich-Peroxidase und alkalischer Phosphatase ausgewählt ist.

6. Interkalationsfarbstoff-Sonde nach einem der vorhergehenden Ansprüche, wobei der Fluoreszenz-Interkalationsfarbstoff aus Acridinorange, Thiazolorange, Ethidiumbromid, SYBR Green I, SYBR Gold, SYBR Safe, EvaGreen, EvaRuby, PicoGreen, SYTO-9, TOTO-1 und YOYO-1 ausgewählt ist.

7. Interkalationsfarbstoff-Sonde nach einem der vorhergehenden Ansprüche, wobei die Interkalationsfarbstoff-Sonde ferner eine zusätzliche DNAbindende Domäne umfasst.

8. Verfahren zum Nachweisen doppelsträngiger DNA in einer Lösung, wobei das Verfahren Folgendes umfasst:
- Inkubieren der Lösung mit dem Interkalationsfarbstoff nach einem der Ansprüche 1 bis 7 und einem geeigneten Substrat für die Lumineszenzdomäne; und
- Nachweisen des Lichts, das von dem Interkalationsfarbstoff abgegeben wird.

9. Verfahren nach Anspruch 8, wobei das Verfahren zum Quantifizieren doppelsträngiger DNA verwendet wird, wobei das Verfahren ferner Folgendes umfasst:
- Nachweisen des Lichts, das von der Lumineszenzdomäne abgegeben wird; und
- Quantifizieren der Menge an doppelsträngiger DNA, basierend auf dem Verhältnis des von dem Fluoreszenz-Interkalationsfarbstoff abgegebenen Lichts und des von der Lumineszenzdomäne abgegebenen Lichts.

10. Verfahren nach Anspruch 8 oder 9, wobei das Verfahren vor, während und/oder nach einer DNA-Amplifikationsreaktion durchgeführt wird.

11. Verfahren nach Anspruch 10, wobei die Amplifikationsreaktion aus Folgendem ausgewählt ist: Polymerase-Kettenreaktion (PCR), Rekombinase-Polymerase-Amplifikation (RPA), schleifenvermittelte isotherme Amplifikation (LAMP= loop mediated isothermal amplification), Nukleinsäuresequenz-basierte Amplifikation (NASBA), Strangverdrängungsamplifikation (SDA= strand displacement amplification), Mehrfachverdrängungsamplifikation (MDA= multiple displacement amplification), Rolling-Circle-Amplifikation (RCA), Ligase-Kettenreaktion (LCR), Helikase-abhängiger Amplifikation (HDA) oder RAM (ramification amplification method).

12. Kit-of-Parts, umfassend die Interkalationsfarbstoff-Sonde nach einem der Ansprüche 1 bis 7 und ein geeignetes Substrat für die Lumineszenzdomäne.

13. Kit-of-Parts nach Anspruch 12, wobei es sich bei dem Substrat um Luciferin handelt, vorzugsweise Furimazin, Firefly-Luciferin, Latia(Schnecken)-Luciferin, Bakterien-Luciferin, Coelentrazin, Dinoflagellat-Luciferin, Vargulin, 3-Hydroxy-Hispidin, Luminol oder H₂O₂.

14. Verwendung der Interkalationsfarbstoff-Sonde nach einem der Ansprüche 1 bis 7 bei einem Verfahren zum Nachweisen doppelsträngiger DNA.

## Revendications

1. Sonde à colorant intercalant pour la détection d'ADN double brin, dans laquelle le colorant comprend une protéine génératrice de luminescence liée à un ou plusieurs colorants fluorescents intercalants.

2. Sonde à colorant intercalant selon la revendication 1, dans laquelle la protéine génératrice de luminescence est une protéine bioluminescente ou une enzyme utilisée pour renforcer la chimioluminescence.

3. Sonde à colorant intercalant selon la revendication 2, dans laquelle la protéine bioluminescente est une protéine ayant une activité luciférase, de préférence choisie parmi : la NanoLuc, la luciférase de luciole, la luciférase de Renilla, la luciférase de Metridia, la luciférase bactérienne, la luciférase de dinoflagellés, la luciférase d'Oplophorus, la luciférase de Gaussia, la luciférase TurboLuc, la luciférase ALuc ou des fragments catalytiquement actifs de celles-ci.

4. Sonde à colorant intercalant selon la revendication 3, dans laquelle la protéine ayant une activité luciférase comprend une séquence d'acides aminés telle que définie par l'un quelconque des identifiants de séquence, SEQ ID, n° 1 à 7.

5. Sonde à colorant intercalant selon la revendication 2, dans laquelle l'enzyme utilisée pour renforcer la chimioluminescence est choisie parmi la peroxydase de raifort et la phosphatase alcaline.

6. Sonde à colorant intercalant selon l'une quelconque des revendications précédentes, dans laquelle le colorant fluorescent intercalant est choisi parmi l'acridine orange, le thiazole orange, le bromure d'éthidium, le SYBR Green I, le SYBR Gold, le SYBR Safe, l'EvaGreen, l'EvaRuby, le PicoGreen, le SYTO-9, le TOTO-1 et le YOYO-1.

7. Sonde à colorant intercalant selon l'une quelconque des revendications précédentes, dans laquelle la sonde à colorant intercalant comprend en outre un domaine de liaison à l'ADN supplémentaire.

8. Procédé de détection d'ADN double brin dans une solution, le procédé comprenant :
- l'incubation de la solution avec la sonde à colorant intercalant selon l'une quelconque des revendications 1 à 7 et un substrat approprié pour le domaine luminescent ; et
- la détection de la lumière émise par le colorant fluorescent intercalant.

9. Procédé selon la revendication 8, dans lequel le procédé est utilisé pour quantifier l'ADN double brin, le procédé comprenant en outre :
- la détection de la lumière émise par le domaine luminescent ; et
- la quantification de la quantité d'ADN double brin sur la base du rapport entre la lumière émise par le colorant fluorescent intercalant et la lumière émise par le domaine luminescent.

10. Procédé selon la revendication 8 ou 9, dans lequel le procédé est mis en œuvre avant, pendant et/ou après une réaction d'amplification de l'ADN.

11. Procédé selon la revendication 10, dans lequel la réaction d'amplification est choisie parmi : la réaction en chaîne par polymérase (PCR), l'amplification par polymérase recombinase (RPA), l'amplification isotherme médiée par les boucles (LAMP), l'amplification basée sur la séquence d'acide nucléique (NASBA), l'amplification par déplacement de brin (SDA), l'amplification par déplacement multiple (MDA), l'amplification en cercle roulant (RCA), la réaction en chaîne par ligase (LCR), l'amplification hélicase-dépendante (HDA) ou la méthode d'amplification par ramification (RAM).

12. Kit de pièces comprenant la sonde à colorant intercalant selon l'une quelconque des revendications 1 à 7 et un substrat approprié pour le domaine luminescent.

13. Kit de pièces selon la revendication 12, dans lequel le substrat est la luciférine, de préférence la furimazine, la luciférine de luciole, la luciférine de Latia (escargot), la luciférine bactérienne, la coelentrazine, la luciférine de dinoflagellés, la varguline, la 3-hydroxy hispidine, le luminol ou H₂O₂.

14. Utilisation de la sonde à colorant intercalant selon l'une quelconque des revendications 1 à 7 dans un procédé de détection d'ADN double brin.
